# EUROPEAN PATENT APPLICATION

(11) **EP 3 479 873 A1**
(43) Date of publication of application: **08.05.2019**
(21) Application number: 17200245.3
(22) Date of filing: 07.11.2017
(51) Int. Cl.: A61N 5/10

(54) **BRACHYTHERAPY QUALITY ASSURANCE USING INVIVO DOSIMETER**

(71) Applicant: Koninklijke Philips N.V., 5656 AE Eindhoven (NL)
(72) Inventor: HAUTVAST, Guillaume Leopold Theodorus Frederik, 5656 AE Eindhoven (NL); BINNEKAMP, Dirk, 5656 AE Eindhoven (NL)
(74) Representative: de Haan, Poul Erik

(57) **Abstract**

A system and a method for brachytherapy treatment are provided, in which a plurality of drive wires are driven inside a plurality of guiding tubes by a drive unit. The plurality of drive wires comprises at least one source wire for driving at least one source of radioactive radiation and at least one measurement wire for driving at least one measurement device for measuring radiation data. The drive unit is controlled by a control unit and is configured to drive the at least one source wire and the at least one measurement wire independently from each other.

## Description

### FIELD OF THE INVENTION

The present invention relates to a system for brachytherapy treatment, in particular High Dose Rate (HDR) brachytherapy treatment, employing Invivo Dosimetry (ID) during the treatment process, a corresponding method and respective computer program. More specifically, the possibility of integrating ID into devices like afterloader devices for quality assurance (QA) is addressed. Even more particularly, the present invention relates to the planning, performing and controlling of the placement and the driving of sources of radioactive radiation and detection devices for treating cancer with brachytherapy.

### BACKGROUND OF THE INVENTION

In (HDR) brachytherapy growths like cancer or tumors are treated by delivering radiation to a target area. Hereby, a remote afterloader is used to move a source of highly radioactive radiation through implanted catheters or channels. The afterloader is controlled by a therapy control system (TCS). More particularly, the TCS loads the brachytherapy plan to instruct the afterloader to drive one or more sources of radioactive radiation through the individual catheters or channels in accordance with said therapy plan. Thus far, such brachytherapy systems are configured to solely perform positioning of the sources of radioactive radiation, while being unaware of the dose they deliver to the target area.

More specifically, in current approaches, the afterloader device merely moves the sources of radioactive radiation to the planned position inside the catheter, without any monitoring of the precise three-dimensional (3D) position of the source of radioactive radiation. Accordingly, there is no position verification with respect to the planned position inside the catheter provided. As a result, the afterloader device cannot verify the actual 3D position and the actual dose that has been delivered to the target area. Furthermore, it is not possible to identify common error scenarios such as the exchanging of two catheters.

Accordingly, within current clinical approaches the actually delivered radiation dose is not known. It is typically assumed that the dose delivered is equal to the (therapy) plan, despite several potential error sources, such as errors arising during the planning process, as well as between planning and delivering the radiation dose. The planning process is hereby limited by positional accuracy for registering the catheters. Further, the planning process assumes a homogenous medium for dose absorption. In addition, even after planning errors may occur. Catheter motion, as well as swelling of the anatomy may cause such errors. A catheter motion may arise due to the connection process, the delivery process or voluntary/involuntary patient motion. Quality assurance (QA) of the dose delivery process using an afterloader system is therefore very limited.

To that end, the introduction of Invivo Dosimetry (ID) allows a more extensive QA. When using ID devices, QA consists of comparing measured dose (rates) with the one anticipated by the therapy plan.

ID involves placing miniature sensors near the target areas. These sensors measure the radioactive dose or the radioactive dose rate during radiation delivery. Thereby, a delivery process should be verified and the actually delivered dose should be reconstructed, whereby common errors may be detected. However, the delivered dose often may exhibit high dose gradients from one position to another. Accordingly, knowledge about the positioning of the measurement devices (dosimeters) with respect to the sources of radioactive radiation and the respective dose (rate) delivered by them is crucial. However, as indicated herein above, position monitoring and verification is currently not available in brachytherapy. As such, QA involving Invivo Dosimetry likewise suffers from certain inaccuracies which may lead to an incorrect assessment of the actually delivered dose (rate).

### SUMMARY OF THE INVENTION

As described herein above, QA of the radiation delivery processes is currently fairly limited. It is therefore an object of the present invention to provide an improved system and a corresponding method for brachytherapy treatment.

A successful QA process depends on the positioning of the measurement devices, which requires a trade-off between several considerations:
- Positioning of measurement devices should be avoided in areas with high spatial gradients in the planned radiation dose distribution. Otherwise, small errors in relative positioning result in large deviations for the measured radiation dose.
- The evolution of the spatial radiation dose gradient over time is affected by the sequence of the positions of the source of radioactive radiation.
- The measurement device may not be sensitive enough to measure the radiation dose or radiation dose rate that is delivered from all source positions. Hence, areas, which exhibit low radiation dose according to the therapy plan should be avoided.
- Side effects from tissue trauma in certain organs should be prevented. Hence, areas where no needles can be placed (so-called no-go zones or no-go areas) should be avoided.
- High confidence QA in crucial areas close to or inside target organs or organs at risk should be prioritised. Hence, placement of sensors or measurement devices - if allowed - should be prioritized for these areas.

This objective is achieved by a system for brachytherapy treatment comprising a drive unit for driving a plurality of drive wires inside a plurality of guiding tubes, the plurality of drive wires comprising at least one source wire for driving at least one source of radioactive radiation and at least one measurement wire for driving at least one measurement device for measuring radiation data and a control unit for controlling the drive unit, wherein the drive unit is configured to drive the at least one source wire and the at least one measurement wire independently from each other.

According to the invention, a system for brachytherapy treatment with one or more drive wires is provided. In this system, one or more sources of radioactive radiation and one or more measurement devices are attached to the drive wires and driven inside respective guiding tubes independently from one another. By driving the at least one source of radioactive radiation and the at least one measurement device independently from each other, the signal-to-noise ratio (SNR) of the measurement devices may be significantly improved. Hereby, it shall be understood that driving the at least one source and the at least one measurement device independently from one another may particularly be performed concurrently, i.e. the driving of the source and the driving of the measurement device is performed at the same time, yet in a different manner, i.e. with different acceleration, different speed, or the like.

In this context, the term brachytherapy treatment refers generally to the treatment of growths (e.g. tumor or cancer) by placing a source of radioactive radiation inside a patient (e.g. human of animal) in areas close to the growth. Such areas may be denoted as target areas or regions of interest (ROIs). Brachytherapy treatment may be performed by placing needles or implants permanently or temporarily inside and/or outside the patient. Such needles or implants may carry sources of radioactive radiation, may support the location measurement of the devices inserted into the patient for the brachytherapy treatment or support the performer of the brachytherapy in other clinical relevant ways. In addition, brachytherapy treatments are often supported by (classical) external radiation therapies for treating growths.

The term brachytherapy treatment may particularly refer to low dose rate (LDR) brachytherapy, pulsed dose rate (PDR) brachytherapy, high dose rate (HDR) brachytherapy or a combination of LDR, PDR or HDR.

HDR brachytherapy treatment comprises the insertion of one or more guiding tubes into a patient. Subsequently, drive wires are driven inside guiding tubes to which sources of radioactive radiation are attached. These sources of radioactive radiation may particularly be highly radioactive sources, such as, for example, Iod-125 or Iridium-192.

In contrast to HDR brachytherapy, LDR brachytherapy treatment comprises the depositing of a less radioactive radiation source for a longer time at a fixed position inside the patient. Accordingly, the source is not newly introduced every treatment session, but rather remains inside the patient and continuously emits radiation in the direction of the target area.

Finally, PDR brachytherapy treatment may be performed as, both, HDR or LDR brachytherapy treatment. During PDR brachytherapy, however, radiation is delivered in pulses.

An improved QA process is implemented by introducing respective measurement devices in addition to the sources into the guiding tubes and by driving the measurement devices and the sources independently by means of a drive unit. In this context, the term drive unit may particularly refer to a unit or module within the system which is configured to drive a plurality of drive wires inside a plurality of guiding tubes. Hereby, the drive unit may typically be controlled by a control unit.

Further, the term measurement device may typically refer to a miniature sensor for Invivo Dosimetry (ID) capable of measuring radiation data occurring at a particular location inside the patient in the proximity of the measurement device. Hereby, the measurement devices may particularly be implemented by one or more of the following technologies:
- Metal oxide semiconductor field effect transistors (MOSFETs).
- Thermoluminescent dosimeters (TLDs).
- Plastic scintillation dosimeters.
- Diamond detectors.
- Radiochromic dosimetry material.

The measurement results acquired by the measurement devices are denoted as radiation data herein. In that context, radiation data may particularly refer to the (local) radiation dose and/or dose rates (doses per time interval). The subsequent transmission of the radiation data, typically to the control unit, may either be performed in a wireless manner or via a wired connection.

Further, the term drive wire refers to a wire for moving a source of radioactive radiation and/or a measurement device to a particular position inside the guiding tube that has been inserted into a patient. In order to establish such movement, the source and/or measurement devices are each attached to respective drive wires. This attachment may be permanent or temporary. In that respect, a drive wire to which a source is attached may typically be referred to as a source wire. Similarly, a drive wire for driving a measurement device may be referred to as a measurement wire. Hereby, drive wires may be suitable for driving both, sources and measurement devices or they may be implemented in a dedicated manner, i.e. the drive wires may also be implemented such as to be suitable for driving a source and/or a measurement device only. In order to obtain sufficient mobility, the drive wires may typically be flexible.

To that end, the drive wires may be implemented solely for driving purposes, i.e. for driving the sources and/or the measurement devices through the guiding tubes. Alternatively or additionally, the drive wires may be implemented as a wire capable of transferring electrical signals from one position along the length of the drive wire through the drive unit to a control unit via a wired connection. The drive wire may particularly be used to transmit the radiation data from the measurement device to the control unit. The signal connection between the drive wires and the control unit may be bidirectional, that is, signals may also be able to be transmitted from the control unit to the measurement device and/or the source mounted on the drive wire.

The drive wires are guided by respective guiding tubes. A guiding tube within this context may refer to a catheter or a channel made of plastic or any other material suitable for being used for medical treatments that is inserted into a patient's tissue in order to move the source and/or the measurement device in the direction of the target area. The ending portion of a guiding tube may be rounded or pointed or may be a needle depending how and where the guiding tube is inserted, i.e. through natural orifices or into a vessel.

Each guiding tube is placed such that the drive wire inside this guiding tube is guided to its predetermined (planned) position. This movement to a predetermined position may particularly be defined in accordance with respective predetermined movement parameters, such as location, velocity and acceleration. The guiding tubes may hereby be attached to openings of an indexer, which may typically be provided as a part of the drive unit.

An indexer may particularly be a module or a unit that may comprise one or more openings through which the drive wires may exit the drive unit. These openings of the indexer may be provided with a labeling in order to perform (visual) indexing of the guiding tubes attached thereto. The indexer may further be connectable to the control unit. The control unit may hereby be provided with the information as to which particular drive wire is driven inside which particular guiding tube, as identified by said labeling.

Additionally or alternatively, the labeling of a drive wire may particularly refer to a relative labeling, wherein the relative labeling indicates a relation between two or more particular drive wires, e.g. one source wire for driving a source of radioactive radiation and one measurement wire for driving a corresponding measurement device to measure the radiation emitted from that source. Accordingly, the indexer may be configured to provide the relation of a measurement device to a source of radioactive radiation, the relation of multiple sources to one or more measurement devices, the relation of multiple measurement devices to one or more sources and/or the relation of multiple sources of radioactive radiation to one another. In summary, the indexer of an embodiment assigns an index (related to the opening and indicated by the labeling) and/or one multi-index to each drive wire (likewise related to the opening and indicated by the labeling).

To that end, the indexer may vary in geometric shape, number of openings, labeling and/or arrangement of the openings. In some embodiments, all variants of the indexer may be attachable to the same drive unit. During system operation, not all openings need to be provided with a guiding tube. Rather, the number of drive wires and the kind of drive wire may be individually selected for the particular purpose according to the treatment plan.

During operation, the sources and/or measurement devices may be driven independently from one another. The driving the at least one source of radioactive radiation and/or the at least one measurement device independently from each other may particularly comprise one or more of the following:
- changing the spatial position of at least one source of radioactive radiation while relative to this source of radioactive radiation the spatial position of at least one measurement device remains unchanged, and/or
- changing the spatial position of at least one measurement device while relative to this measurement device the spatial position of at least one the sources of radioactive radiation remains unchanged, and/or
- changing the spatial position of at least one source of radioactive radiation while relative to this source of radioactive radiation also changing the spatial position of at least one measurement device, whereby the changing may particularly be performed differently between the at least one source of radioactive radiation and the at least one measurement device.

In that respect, changing the spatial position may comprise a changing of the 3D position and/or a changing of the velocity and/or a changing of the acceleration of a source and/or a measurement device.

The system further comprises a control unit, which may e.g. be implemented as part of a therapy control system (TCS). Hereby, respective measurement values may be obtained by (and stored in) the control unit. Prior to the actual brachytherapy treatment, a therapy plan is derived individually for each patient. To that end, deriving a therapy plan may particularly comprise determining the amount of dose to be delivered to the target area. The therapy plan may subsequently be provided to the control unit, which may then derive, based on the therapy plan, how many sources of radioactive radiation shall be used and where the sources of radioactive radiation shall be positioned. Further, the control unit may derive the number and positions of the measurement devices. The control unit may use this information to control the drive unit to drive the drive wires to the respective positions determined for each one of the sources and/or measurement devices. Finally, the control unit may also be used to compare measured dose and/or dose rate to the dose and/or dose rate according to the therapy plan.

The operation of the control unit may be performed automatically according to the therapy plan, by an interplay of an automatic control according to the therapy plan and a manual control by a user and/or by a manual control. Hereby, manual control may typically be prioritized over automatic control.

To that end, the control unit may particularly be connected to a manual input means for input provided by a user. More specifically, the manual input means may be provided to input information and/or control commands for the desired 3D dose distribution. These inputs may then be transmitted to the control unit and/or the drive unit. Further, the control may also be performed remotely in order to obtain sufficient distance between the user and the source of radioactive radiation, thereby improving user security.

The new system allows dynamic control of the delivered radiation dose and the locations of the measurement devices and/or sources of radioactive radiation during dose delivery. As a result, more extensive treatment quality assurance (QA) is achieved.

The system may particularly be implemented as a remote afterloader system equipped with a therapy control system (TCS). The TCS may be equipped with a processing software which is capable of verifying the delivered radiation dose and/or radiation dose rate. Such system may be capable of reconstructing the actually delivered radiation dose dynamically and in real-time.

Alternatively or additionally, an automated method is proposed which is capable of optimizing the location for placing the measurement devices and/or the sources of radioactive radiation. The automated method may be extended by including simultaneous optimization of the measurement device, the source of radioactive radiation, the needle and/or implant sequence all of which are used in support of QA. The described method may be integrated in a TCS or in a brachytherapy planning, control and verification system.

In some embodiments, the at least one source wire and/or the at least one measurement wire are driven inside at least one hybrid guiding tube

Typically, the size of a source of radioactive radiation may differ from the size of a measurement device. However, in some embodiments, the guiding tubes may be suitable for accommodating both, a source as well as a measurement device. These guiding tubes are called hybrid guiding tubes. For these hybrid guiding tubes, a coordinated driving of the drive wires is necessary in order to prevent a source wire having a source attached thereto and a measurement wire carrying a measurement device entering the same hybrid guiding tube at the same time. Thus, the drive unit and the control unit may be adjusted to implement a respective collision control.

In some embodiments, the plurality of guiding tubes comprises at least one source guiding tube and at least one measurement guiding tube, wherein the at least one source wire is driven inside at least one source guiding tube and the at least one measurement wire is driven inside at least one measurement guiding tube.

In some embodiments, the guiding tubes may not be suitable for both, the size of a measurement device and the size of a source. In these cases, dedicated guiding tubes, such as dedicated channels or dedicated catheters have to be provided. In this context, a measurement guiding tube may refer to a guiding tube dedicated to guiding measurement wires for moving measurement devices, whereby no source wire may be driven inside the measurement guiding tube. In turn, the term source guiding tube may refer to a guiding tube dedicated to guiding source wires for moving sources of radioactive radiation. Hence, all or some of the one or more sources of radioactive radiation are attachable to the at least one source wire and are adapted to be driven inside one or more source guiding tubes and all or some of the one or more measurement devices are attachable to the at least one measurement wire and are adapted to be driven inside one or more measurement guiding tube.

Depending on the therapy plan, the number, the physical size and the radiation intensity of the sources and/or the number, physical size and/or strength of the measurement devices may differ. The radiation intensity of the sources of radioactive radiation typically refers to the amount of radiation emitted by a source and is typically measured in Becquerel (Bq), whereas the dose delivered to the tissue may typically be measured in Gray (Gy) or Sievert (Sv). The strength of the measurement device may be defined as the susceptibility and the accuracy of a measurement device for the radiation measurement.

In some embodiments, driving the at least one source wire and the at least one measurement wire independently from each other comprises moving the at least one source of radioactive radiation driven by the at least one source wire along a plurality of source positions inside at least one guiding tube of the plurality of guiding tubes, while positioning the at least one measurement device driven by the at least one measurement wire at a measurement position that is fixed inside the at least one guiding tube.

Alternatively or additionally, driving the at least one source wire and the at least one measurement wire independently from each other comprises moving the at least one source of radioactive radiation driven by the at least one source wire along a plurality of source positions inside at least one guiding tube of the plurality of guiding tubes, and moving the at least one measurement device driven by the at least one measurement wire along a plurality of measurement positions along a longitudinal axis of the at least one guiding tube.

Alternatively or additionally, driving the at least one source wire and the at least one measurement wire independently from each other comprises positioning the at least one source of radioactive radiation driven by the at least one source wire at a source positions that is fixed inside at least one guiding tube of the plurality of guiding tubes, and moving the at least one measurement device driven by the at least one measurement wire along a plurality of measurement positions along a longitudinal axis of the at least one guiding tube.

As indicated herein above, some or all drive wires may be driven independently from one another. Hereby, the drive wires are moved along a longitudinal axis of the guiding tube. In this context it shall be understood that the term independent driving of the drive wires may also refer to a situation in which all or some of the drive wires' position remain unchanged or in which all or some of the drive wires are driven at the same time (a) with the same speed and the same acceleration, (b) different speed and the same acceleration, (c) same speed and different acceleration or (d) different speed and different acceleration. In total, the way of the moving in space of one drive wire does not affect the way of moving of any of the other drive wires in space.

Accordingly, in some embodiments, at least one source may be moved along a plurality of source positions along a longitudinal axis of a guiding tube by a respective drive wire while at least one corresponding measurement device that is arranged to measure the radiation emitted by the source is maintained at a fixed position. The term fixed position hereby refers to the situation in which the measurement device is driven to a particular measurement position inside the guiding tube and remains at this position during the brachytherapy treatment session or for a predefined time interval during said brachytherapy treatment session. By providing the measurement device at a fixed position, a simplification of the control mechanism during measurement may be achieved. In that respect, when providing the measurement device at a fixed position, a fixed position with sufficient dose should be selected. Further, it should be deferred from selecting a position with strong dose gradients.

Accordingly, the at least one measurement device, alternatively or in addition to keeping it at a fixed position, e.g. for a particular time interval, may also be moved during the brachytherapy treatment session. Hereby, the at least one measurement device is particularly driven by the measurement wire along a plurality of measurement positions aligned along a longitudinal axis of the guiding tube into which the measurement device has been inserted. In this context, the term measurement position refers to a position at which at least one radiation data value is acquired by the measurement device.

In some embodiments, it may also be possible to keep at least one source of radioactive radiation at a fixed position while driving the related measurement device along a plurality of measurement positions along the longitudinal axis of the guiding tube. In this situation, the at least one source is driven, by a respective source wire, to a pre-defined source position and maintained at this position during the brachytherapy session. The embodiment in which the measurement device is moved and, therefore, acquires a plurality of radiation data values at a plurality of measurement positions, while the source is maintained at a fixed source position allows for a straightforward measurement of the dose distribution along the target area.

In some embodiments, the controlling, by the control unit, further comprises receiving source position data indicating at least one source position of the at least one source of radioactive radiation driven by the at least one source wire and receiving measurement position data indicating at least one measurement position of the at least one measurement device driven by the at least one measurement wire.

Obtaining definite knowledge about the position of the one or more sources and/or the one or more measurement devices may be beneficial. Accordingly, in some embodiments, source position data indicative of the position of at least one source during brachytherapy treatment may be provided to the control unit. In this context, the term source position data may refer to any kind of data from which the position of the source, in particular inside the guiding tube may be derived. In some embodiments, the source position data may be provided to the control unit from a respective tracking unit configured to track the source position of the source. This tracking may be performed during the driving of the source inside the guiding tube. Hereby, the tracking may be performed while moving the source in the direction of the target area. Further, the tracking may, alternatively or additionally, be performed during the treatment process itself, i.e. while the source is arranged to irradiate the target area.

Alternatively or additionally, measurement position data indicative of the position of at least one measurement device during the treatment may be provided to the control unit. The term measurement position data may particularly refer to any kind of data from which the position of the measurement device, in particular inside the guiding tube, may be derived. In some embodiments, the measurement position data may also be obtained using established tracking methods, such as using a fluoroscopic recording of the position of the source and/or the measurement device.

In some embodiments, at least one source tracking device is arranged in close proximity to the at least one source. The source position data is then derived from source tracking information obtained by the at least one source tracking device. Further, at least one measurement tracking device is arranged in close proximity to the at least one measurement device, whereby the measurement position data is derived from measurement tracking information obtained by the at least one measurement tracking device.

In this context, the term close proximity refers to a region which is close enough to the source and/or measurement device that the source and/or measurement device may be tracked by the tracking device. In some embodiments, the tracking device may be arranged adjacent to the source and/or measurement device on the respective drive wire. In some embodiments, the tracking device may be attached to the source and/or the measurement device. In some embodiments, the tracking device may be incorporated into the source and/or the measurement device.

A tracking device may particularly be implemented as a tracking sensor. Hereby, a source tracking device may particularly refer to a tracking device for tracking at least one source and a measurement tracking device may refer to a tracking device for tracking at least one measurement device. The tracking devices may particularly be configured to acquire tracking information indicating the three-dimensional location of a tracked source and/or a tracked measurement device inside the patient's body.

In some embodiments, an external tracking unit may further be implemented. Such an external tracking unit may particularly be a field generator for generating a magnetic field. The thus generated magnetic field induces a magnetic field in the tracking sensors thereby allowing a tracking of these sensors. Other tracking technologies such as, for example, fiber optic tracking technologies, radiography technologies, CT scan and/or magnetic resonance imaging may also be realized for one embodiment of the system.

The tracking information obtained by the source tracking device may be used to the derive source position data indicative of the position of the source. This may for example be performed by collecting a plurality of tracking values and deriving, based on said tracking values, the source position of the source during the entire driving sequence. In a similar manner, the tracking information obtained by the measurement tracking device may be used to derive measurement position data.

In some embodiments, the source tracking information, the measurement tracking information, the source position data and/or the measurement position data are used along with the radiation data may be used by the control unit, by a computing unit and/or by the TCS to compute a three-dimensional radiation dose distribution.

In some embodiments, the system may be implemented such that a desired position of at least one source and/or at least one measurement device may be input into the system by a user. The source position data and/or measurement position data may then be used to drive the source and/or the measurement device to the respective position. Hereby, the source position data and/or measurement position data may particularly be computed in real time and, thus, the actual source position and/or measurement position may be obtained.

In some embodiments, the controlling by the control unit further comprises receiving the radiation data measured by the at least one measurement device at the at least one measurement position and deriving, based on the radiation data, dose distribution data of the dose delivered by the at least one source of radioactive radiation. According to an even further embodiment, the controlling, by the control unit, further comprises comparing the dose distribution data with respective predicted dose distribution data predicted based on a therapy plan, and triggering at least one indication if the dose distribution data and the predicted dose distribution data deviate from one another by a pre-defined tolerance.

In some embodiments, the radiation data measured by the at least one measurement device is used to compute respective dose distribution data of the dose distribution in the target area. In this context, it shall be understood that the term dose distribution data may encompass the dose determined for a single position. Additionally or alternatively, the dose distribution data encompasses the distribution of the dose for a plurality of positions in the target area, which have been irradiated.

In some embodiments, a predicted dose distribution is anticipated by the therapy plan. In this context, it shall be understood that the term therapy plan refers to a plan for brachytherapy treatment as derived for a patient. The therapy plan for brachytherapy treatment may particularly be determined before the brachytherapy treatment (pre-determined plan) or may be adapted during the brachytherapy treatment (re-scheduled plan). Such adaption may be necessary if e.g. error scenarios occur or if due to measured data an adaption of the brachytherapy treatment becomes necessary. The therapy plan may be derived in a planning unit. The planning unit may communicate with the control unit via a wired connected and/or wireless. The therapy plan may particularly comprise the predicted dose distribution, the time frames for said predicted dose distribution and/or a predicted dose rate, as well as the distribution thereof, that should be applied to growths in the patient.

It shall be understood that the predicted dose distribution may be compared to the dose distribution determined from the measurements by the measurement device. This comparison between the measured dose distribution and the predicted dose distribution, performed by comparing both respective data sets representing the measured and predicted dose distribution, may particularly allow to identify specific error scenarios and/or a situation in which the above-mentioned adaption of the therapy plan may be necessary.

More specifically, it may be determined from such a comparison, that the measured and the predicted dose (distribution) deviate from one another. Such a deviation may be an indication for a misplacement of the source of radioactive radiation and/or of the measurement device. As an example, such a deviation may be indicative of the guiding tubes or channels having been switched, so that the source is currently driven inside the wrong guiding tube (e.g. the guiding tube supposed to be used for the measurement device or an entirely different guiding tube). As a further example, it may be derived from the comparison that the radiation emitted by the source is absorbed stronger or is not absorbed as well as planned, thereby requiring an adaption of the therapy plan.

When performing such an error detection by means of a comparison between measured and predicted dose distribution, measurement and calculation errors in deriving the measured dose distribution should be considered. That is in reality, the measured dose distribution will naturally deviate from the predicted dose distribution by a respective distribution delta.

This distribution delta may be used as a threshold value for the error detection. If the distribution delta is inside a pre-defined tolerance the treatment continues without deviating from the therapy plan. If the distribution delta is not inside a pre-defined tolerance the treatment may continue with an adapted or re-scheduled therapy plan and/or may indicate an error.

In some embodiments, this indication may particularly be performed by triggering at least one indication. Such an indication may particularly be an audible or visible alarm. This alarm may show that the distribution delta is not inside a pre-defined tolerance. The user may then decide how to proceed or if the treatment should be cancelled.

In some embodiments, the controlling, by the control unit, further comprises optimizing at least one measurement position of the at least one measurement wire on the basis of predicted dose distribution data obtained from a predicted dose distribution anticipated by the therapy plan. In some embodiments, the optimizing of the at least one measurement position further comprises identifying a plurality of candidate positions for the measurement device, predicting, based on the therapy plan, the predicted dose distribution for each of the plurality of candidate positions in order to obtain the dose distribution data, and determining, from the plurality of candidate positions, the at least one measurement position using a penalty method applied to the predicted dose distribution data.

In some embodiments, position optimization of the measurement position may be performed based on the predicted dose distribution as derived from respective predicted dose distribution data. That is, a measurement position may be selected such as to lie in a region in which the best measurement results may be obtained. In some embodiments, this optimization encompasses the deriving of one or more positions which may be potential candidates for placing the measurement devices and determining the dose distribution for each of these positions. A measurement position may then be determined by applying a penalty method using the predicted dose distribution data.

More particularly, in some embodiments, a planning unit is used which computes the optimal positioning of at least one measurement device. For the optimization, the above-mentioned candidate positions for the measurement devices in or near the target area, regions of interest or organs at risk are identified which are not yet taken by the needles, implants or guiding tubes used for therapy delivery, and which are not in a no-go zone.

Using the tracks resulting from the plurality of candidate positions for the measurement devices, the module then computes the predicted dose or dose rate for each candidate position along said track. Hereby, a numerical optimization employing a penalty method is used. Accordingly, the requirements for the measurement devices are translated in a set of representative penalty functions. These penalty functions can be used in a (weighted) sum to formulate the objective function that is to be optimized by the optimization software.

In this context, the following penalty functions may particularly be used for optimizing the measurement position of at least one measurement device.

Positioning measurement devices in areas with high spatial gradients in the predicted dose distribution are penalized by computing the spatial gradient amplitude in the final plan at each candidate position. If a higher penalization is required, one might opt for using the squared gradient magnitude, or consider the sum or maximum of the x, y, and z components of the spatial gradient in the final plan.

Positioning measurement devices in areas with low dose or dose rate in the predicted dose distribution are penalized by computing the difference between a minimal acceptable level and the predicted level of the dose or dose rate. Alternate formulations may substitute this difference in a (single-sided) quadratic or higher order polynomial, in an exponential function, or in a Heavyside function.

Measurement positions close to regions of interest are prioritized by computing the distance between a candidate position and such a region of interest. Alternate formulations may substitute this distance in a (single-sided) quadratic or higher order polynomial, in an exponential function, or in a Heavyside function.

In addition, variations on this penalty function may be formulated by computing the distance to local minima/maxima in the planned dose distribution to propose measurement positions such that the dose or dose rate is measured where it matters most.

The penalty functions may be joined in an objective function using a weighted sum. The weightings may be assigned (automatically and/or by the user) to alter the balance between the individual aspects in the problem.

The core optimization method minimizes the complete objective function by moving the measurement devices through the solution space. This core method can rely on any numerical optimization technique. An optimization method in particular, it can be implemented using any of the following:
- Greedy optimization
- Gradient-based optimization (e.g. Powel's search, conjugate gradient approaches, or more advanced (1-)BFGS approaches).
- Stochastic optimization, (e.g. simulated annealing or genetic algorithms).

In support of being able to identify errors scenarios, another penalty function may be introduced, computing for example the correlation of the predicted dose or dose rate signal that is anticipated according to the plan with the dose or dose rate signal in case the guiding tubes are connected wrongly. This will drive the solution towards a location where the measurement changes most if an error occurs. Again, alternate formulations may substitute the correlation in a (single-sided) quadratic or higher order polynomial, in an exponential function, or in a Heavyside function. In addition, instead of using correlation measures, on might rely on simple difference measures or more advanced mutual information measures.

According to a further aspect, a method for brachytherapy treatment is provided, the method comprising the steps of: driving, by a drive unit controlled by a control unit, a plurality of drive wires inside a plurality of guiding tubes, the plurality of drive wires comprising at least one source wire for driving at least one source of radioactive radiation and at least one measurement wire for driving at least one measurement device for measuring radiation data, wherein the drive unit is configured to drive the at least one source wire and the at least one measurement wire independently from each other.

In a further aspect, a computer program for controlling the above-described system is provided, which, when executed by a processing unit, is adapted to perform the method steps according to the invention. In an even further aspect, a computer-readable medium is provided having stored thereon the above-cited computer program.

It shall be understood that the system for brachytherapy treatment may be implemented by means of a processing unit. Hereby, the drive unit, the control unit, planning unit, the alarm unit, the visualization unit, the manual control unit, the sending unit and the external measurement unit may be implemented as modules in the processing unit. The functionality of these modules may in particular be implemented by means of a respective algorithm. This algorithm may in particular be implemented using a machine learning algorithm implemented on said processing unit including said modules.

It shall be understood that the system of claim 1, the method of claim 13, the computer program of claim 14, and the computer-readable medium of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically illustrates a system for high dose rate brachytherapy treatment according to an embodiment.
Fig. 2 schematically illustrates the indexer 107 with attached guiding tubes 123.
Fig. 3 schematically illustrates a method for high dose rate brachytherapy according to an embodiment.

### DETAILED DESCRIPTION OF EMBODIMENTS

The illustration in the drawings is schematically. In different drawings similar or identical elements are provided with the same reference numerals.

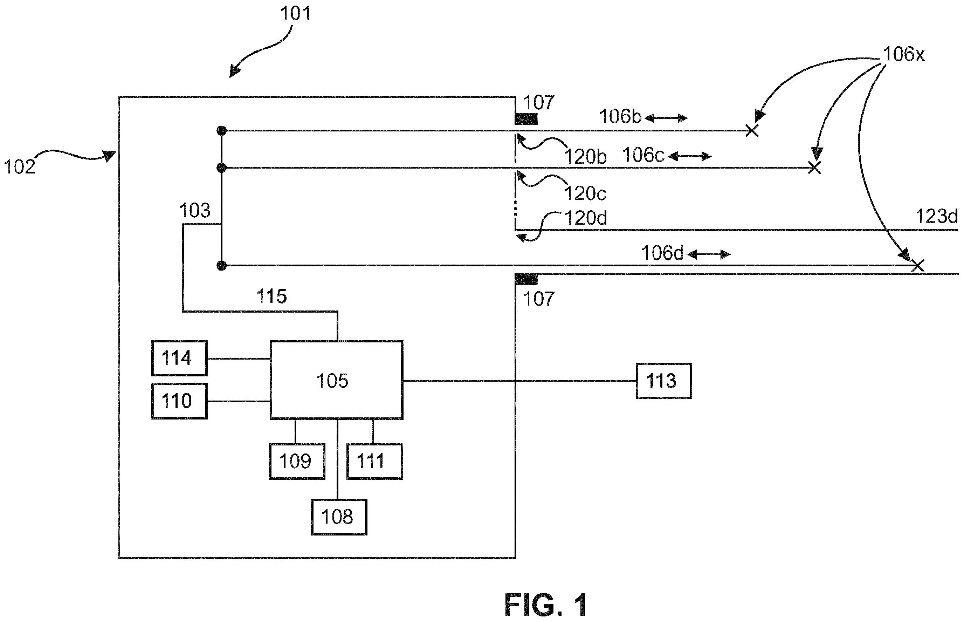

Fig. 1 schematically represents a system 101 for high dose rate brachytherapy treatment. The system 101 comprises a drive unit 102. The drive unit 102 may be an afterloading device or an afterloader. The drive unit 102 is configured to drive one or more drive wires 106. In the exemplified embodiment, three drive wires 106b, 106c and 106c are visualized. The drive wires 106b, 106c and 106d are individually attached to one or more mountings 103 in the drive unit 102. The drive wires 106b, 106c and 106d exit the drive unit 102 through at least one opening 120. The relevant length of one particular drive wire 106 may be defined as the length of the drive wire between the mounting 103 and the ending portion 106x of the drive wire. Driving of one drive wire 106 comprises varying the length of the drive wire 106, that is, changing the spatial position of the ending portion 106x of the drive wires 106b, 106c and 106c outside the drive unit or moving the ending portion 106x of the drive wire back through the respective opening 120b, 120c, 120d into the drive unit 102. The mounting 103 may comprise a respective drive mechanism for mechanically driving the drive wires 106b, 106c, 106d. This driving may particularly be performed concurrently for all or at least a subset of the drive wires, i.e. all or at subset of the drive wires are driven at the same time, but in a manner independent of each other.

Hence, at least two of the drive wires 106b, 106c and 106d may be driven independently from one another. That is, the length of one drive wire 106b may remain unchanged while at the same time the length of another drive wire 106c changes, or, alternatively or additionally, the length of both drive wires 106b and 106c changes with differing speed and/or acceleration. The directions of spatial movement of the drive wires 106b, 106c and 106d are schematically visualized by the straight arrows ↔ in Fig. 1.

The system 101 further comprises a control unit 105 which is connected via a connection 115 to the mounting 103 to control the driving of the drive wires 106b, 106c, 106d. The connection 115 may particularly be a signal connection for sending control signals to the mounting 103 of the drive unit 102. These control signals cause the drive unit 102 to drive the drive wires, via the mounting 103, to their respective positions as indicated by the control unit. To that end, the control unit 105 may particularly be or be connected to a therapy control system (TCS) generating the control signals in accordance with the desired therapy. In the exemplary embodiment according to Fig. 1, the control unit 105 is implemented as part of the drive unit 102. It shall be understood that the control unit 105 may alternatively be a separate entity external to the drive unit 102.

The control unit 105 may further be communicatively connected to an external tracking unit 113, which is used for deriving source tracking information for the sources and measurement tracking information for the measurement devices. In the particular embodiment of Fig. 1, the tracking unit 113 may belong to an electromagnetic tracking system. More specifically, the tracking unit 113 may be a field generator generating a magnetic field which induces a magnetic field in sensors attached to the drive wires 106b, 106c and 106d in close proximity to the sources and/or measurement devices to be tracked. The tracking information obtained by the sensors and the tracking unit 113 may then be communicated to the control unit 105. The control unit 105 uses the source tracking information to derive respective source position data and the measurement tracking information to derive measurement position data.

The system 101 further comprises a planning unit 114 which may also be in communicative connection to the control unit 105. The planning unit 114 is configured to develop a therapy plan. The provisions for the planning unit 114, such as radioactive doses, radiation time and duration, number and kinds of used active sources or the like, are input into the planning unit 114 by a user via a respective user input means that is part of the planning unit 114. The planning unit 114 may further provide, to the control unit 105, information about the therapy plan, such as the positions to which the drive unit 102 has to drive each one of the sources and each of the measurement devices and/or a predicted dose distribution for a particular measurement position relative to a particular source distribution et cetera.

To that end, the control unit 105 may be configured to use this information to detect deviations from the therapy plan, such as deviations between the data indicative of the actually measured dose distribution and the data indicative of the predicted dose distribution. In that respect, the deviation may particularly be defined by a respective threshold value. This threshold value may particularly be configurable by a user in accordance with the requirements of the therapy, the target organs and/or the fractional schemes. For this purpose, the control unit 102 further comprises a user interface 111 that may particularly be connected to the drive unit 105. The user may configure the drive unit 105 via the user interface 111. This configuration may particularly encompass the configuration of the threshold value. Further configurations may likewise be made by the user. Finally, the user interface 111 may also be used for manual control of the drive unit 105 where manual control is required.

The threshold value may particularly be configured to lie in the range between 1% and 20% deviation, even more particularly between 1% and 5% deviation. In some embodiments, when comparing the dose distribution, a common threshold for a deviation may even more particularly be configured to be a deviation in the range of less than 1%. In some embodiments, it may be determined, e.g. by a respective user configuration, that the measured and predicted dose shall not deviate at all.

The control unit 105 may further be configured to derive the cause for such deviations, such as an error according to which two guiding tubes have been physically exchanged during the connection to the drive unit 102. Likewise, unplanned motions of the guiding tube due to the connection process, the delivering process, voluntary and involuntary motion of the patient, swelling of the anatomy or like issues are possible causes for deviations.

Depending on the kind of deviation and the determined cause for said deviation, the control unit 105 may provide an indication to a user. For this purpose, the system may further comprise an alarm unit 110 that may typically be connected to the control unit 105. The alarm unit 110 may be configured to display a visible and/or provide an audible and/or a tactile alarm, such as a vibrational indication, that an error and/or a deviation from the therapy plan has occurred. Preferably, the alarm unit 110 may also visualize a classification of the error or deviation that has been detected. To that end, the alarm unit 110 may particularly comprise a display screen that is able to display text representing an indication of the kind of error that has occurred and further providing a help display indicating the steps to be taken in order to eliminate that error.

The control unit 105 may further be connected to a visualization unit 109. The visualization unit 109 may be able to provide a graphical representation of the radiation data measured by the measurement devices and/or the dose distribution data derived by the control unit 105 from the radiation data. The visualization unit 109 may further be able to present the graphical representation to a user of the drive unit 102. Examples for relevant information that may be visualized in the graphical representation provided by the visualization unit 109 may for example relate to the radiation data obtained by the measurement devices, the dose distribution data derived therefrom, to source and measurement position information or the like. The visualization unit 109 may also visualize the history of the current treatment and/or the upcoming steps for the current treatment. The visualization unit 109 may further visualize the therapy plan, e.g. along with the predicted dose distribution. Further, the visualization unit 109 may provide information for the user that are necessary for the manual control of the drive unit 102, which may be used to replace or extend the automatic control by the control unit 105.

Finally, the system 101 also comprises a transmission unit 108 which allows the radiation data, source position information and/or measurement position information received at the control unit 105 to be communicated to external devices. Such external devices may be devices that are employed in addition to HDR brachytherapy devices for treating growths, such as external irradiation devices, in order to obtain sufficient quality assurance (QA) of the overall treatment process, or to other relevant working devices such as portable computers and/or fixed computers.

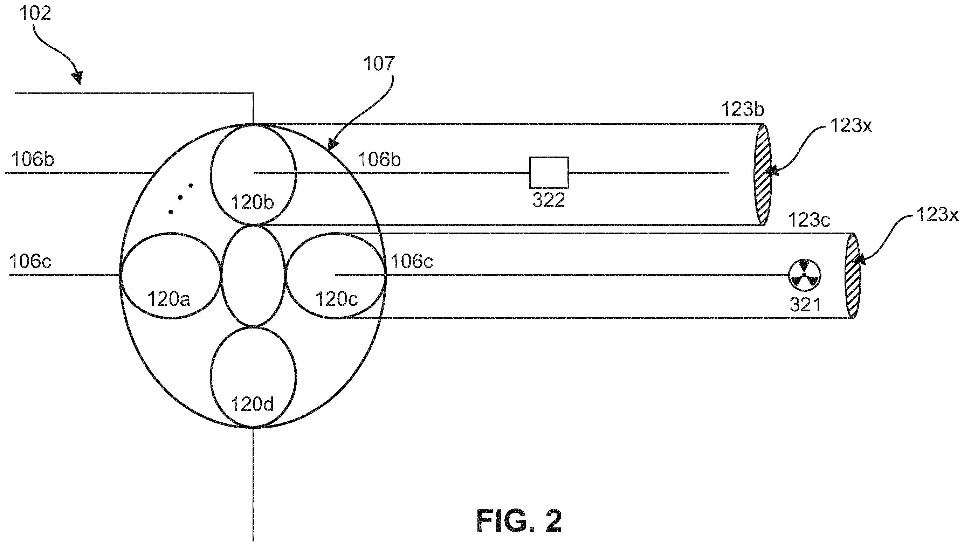

As schematically represented in Fig. 2, at least one measurement device 322 for acquiring radiation data may be attached to a drive wire 106b and at least one source of radioactive radiation 321 may be attached to a drive wire 106c.

To guide the drive wires 106b and 106c, guiding tubes 123b and 123c may be attached to openings 120b and 120c of an indexer 107, further comprising openings 120a and 120d, to which no guide wires are connected in this embodiment. The indexer 107 may be part of the drive unit 102 or may be implemented as a separate entity. The indexer 107 may comprise the openings 120 through which the drive wires 106b and 106c exit the drive unit 102. More particularly, the measurement drive wire 106b may exit the drive unit 102 and enter the guiding tube 123b through opening 120b. Likewise, the source drive wire 106c may exit the drive unit 102 and enter the guiding tube 123c through opening 120c. Guiding tubes 123b and 123c are tightly connected to openings 120b and 120c, respectively, such that drive wires 106b and 106c exiting the drive unit 102 may instantly enter the guiding tubes 123b and 123c. The guiding tubes 123b, 123c are provided as having particular length before being terminated by respective ending portions 123x.

In the exemplary embodiment according to Fig. 2, the guiding tubes are embodied as dedicated guiding tubes, i.e. guiding tubes that may only be used to guide measurement wires for driving measurement devices or source wires for driving sources. Accordingly, guiding tube 123b corresponds to a measurement guiding tube, in which measurement device 322 may be driven by measurement wire 106b. Guiding tube 123c corresponds to a source guiding tube, in which source 321 may be driven by source wire 106c.

In alternative embodiments, however, the guiding tubes may be configured as hybrid guiding tubes and each guiding tube may be suitable for guiding both, source wires for driving sources or measurement wires for driving measurement devices, whichever is desired for a particular brachytherapy session.

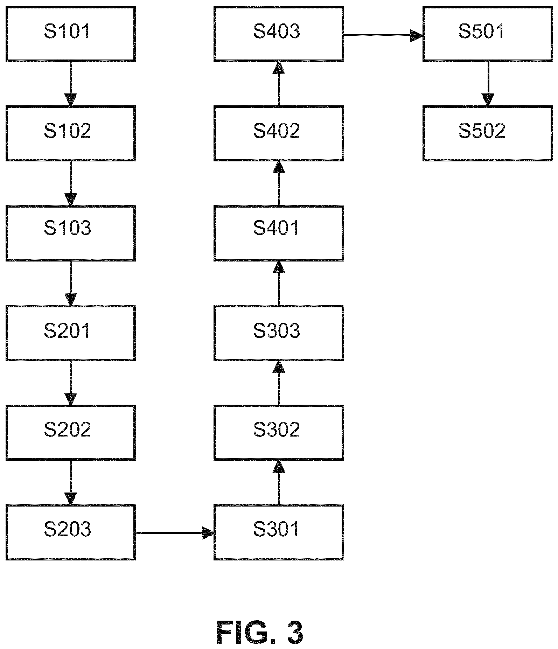

Fig. 3 schematically represents a method for brachytherapy treatment using Invivo Dosimetry according to an embodiment. In step S101, a plurality of candidate positions for positioning the measurement devices is identified based on the treatment plan. Hereby, it is particularly determined which positions are sufficiently close to the target area, have not been planned to be used for sources and are not in a no-go zone. Subsequently, in step S102, the predicted dose distribution along the track formed by these candidate positions is determined based on the planned source positioning and radiation intensity of the sources. In step S103, at least one measurement position for at least one measurement device is determined, optionally by
- Penalizing positioning in areas with high spatial gradients in the planned dose distribution,
- Penalizing positioning in areas with low dose or dose rate in the planned dose distribution, and
- Prioritizing positioning close to regions of interest.

In step S201, guiding tubes for the measurement devices are inserted to encompass the thus determined measurement positions. Further, the guiding tubes for the sources are inserted. In the embodiment according to Fig. 3, the guiding tubes for the sources are dedicated source guiding tubes and the guiding tubes for the measurement devices are dedicated measurement guiding tubes. In step S202, a measurement device is driven inside a respective guiding tube by means of a measurement wire to the optimized measurement position. In step S203, the source is driven inside a different guiding tube by means of a source wire to a respective (planned) source position.

In step S301, measurement tracking information is obtained and measurement position data is derived therefrom and provided to the control unit. In step S302, source tracking information is acquired and source position data is derived from the source tracking information and transmitted to the control unit. These control unit verifies the source position and the measurement position based on the respective position data in step S303 and determines that the source and the measurement device have been correctly positioned by the drive unit.

In step S401, the control unit further receives the radiation data acquired by the measurement devices. The control unit uses the radiation data, and optionally the measurement position data, to determine respective dose distribution data indicative of the radiation dose to the target area in step S402. In step S403, the control unit compares the measured dose distribution data with the predicted dose distribution data as derived from therapy planning and detects a deviation between the values of the measured dose distribution data and the predicted dose distribution data. Further, the control unit determines that the deviation is above a pre-determined threshold value. More particularly, in the exemplary embodiment of Fig. 3, the control unit determines that the deviation is more than 1%.

Accordingly, the control unit, in step S501, triggers an indication, in this particular case an audible and visible alarm by the alarm unit that a deviation from the plan has occurred. The control unit then determines, based on the therapy plan and a respective error cause register, what may be cause for the deviation. In the exemplary embodiment of Fig. 3, the control unit determines that the source guiding tube and the measurement guiding tube of a source/measurement device combination have been physically exchanged, i.e. incorrectly connected to the drive unit. In step S502, the control unit therefore triggers the visualization unit to visualize the cause for the deviation to a user.

Although the above-described embodiments relate to methods for high dose rate (HDR) brachytherapy, it shall be understood that the invention is not limited to HDR brachytherapy, but may also be applied to low dose rate (LDR) brachytherapy or pulsed dose rate (PDR) brachytherapy.

Further, it shall be understood that, although in the above-mentioned embodiments, only a single measurement wire and a single source wire are represented, a plurality of measurement wires and sources wires may be used in accordance with the invention.

It shall further be understood that, although in the above-mentioned embodiments, the control unit comprises the drive unit, the alarm unit, the user interface et cetera, each of these units may be provided as a separate entity, each of the entities being communicatively connectable to one another.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like optimization of positions, driving of the drive wires, measuring doses, dose distributions and/or source positions, manual and/or automatic controlling, planning of the (therapy) plan, indicating by an audible and/or tactile and/or visible alarm, visualization, sending of radiation data and/or tracking information and/or position data, et cetera performed by one or several units or devices can be performed by any other number of units or devices. These procedures in accordance with the invention can hereby be implemented as program code means of a computer program and/or as dedicated hardware.

A computer program may be stored and/or distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a system for brachytherapy treatment comprising a drive unit for driving a plurality of drive wires inside a plurality of guiding tubes, the plurality of drive wires comprising at least one source wire for driving at least one source of radioactive radiation and at least one measurement wire for driving at least one measurement device for measuring radiation data, and a control unit for controlling the drive unit, wherein the drive unit is configured to drive the at least one source wire and the at least one measurement wire independently from each other.

## Claims

1. A system for brachytherapy treatment comprising
- a drive unit for driving a plurality of drive wires inside a plurality of guiding tubes, the plurality of drive wires comprising at least one source wire for driving at least one source of radioactive radiation and at least one measurement wire for driving at least one measurement device for measuring radiation data; and
- a control unit for controlling the drive unit;
wherein the drive unit is configured to drive the at least one source wire and the at least one measurement wire independently from each other.

2. The system according to claim 1,
wherein the at least one source wire and/or the at least one measurement wire are driven inside at least one hybrid guiding tube

3. The system according to claim 1, wherein
the plurality of guiding tubes comprises at least one source guiding tube and at least one measurement guiding tube, wherein the at least one source wire is driven inside at least one source guiding tube and the at least one measurement wire is driven inside at least one measurement guiding tube.

4. The system according to claim 1, wherein driving the at least one source wire and the at least one measurement wire independently from each other comprises
- moving the at least one source of radioactive radiation driven by the at least one source wire along a plurality of source positions inside at least one guiding tube of the plurality of guiding tubes; and
- positioning the at least one measurement device driven by the at least one measurement wire at a measurement position that is fixed inside the at least one guiding tube.

5. The system according to claim 1, wherein driving the at least one source wire and the at least one measurement wire independently from each other comprises
- moving the at least one source of radioactive radiation driven by the at least one source wire along a plurality of source positions inside at least one guiding tube of the plurality of guiding tubes; and
- moving the at least one measurement device driven by the at least one measurement wire along a plurality of measurement positions along a longitudinal axis of the at least one guiding tube.

6. The system according to claim 1, wherein driving the at least one source wire and the at least one measurement wire independently from each other com-prises
- positioning the at least one source of radioactive radiation driven by the at least one source wire at a source positions that is fixed inside at least one guiding tube of the plurality of guiding tubes; and
- moving the at least one measurement device driven by the at least one measurement wire along a plurality of measurement positions along a longitudinal axis of the at least one guiding tube.

7. The system according to claim 1, wherein the controlling, by the control unit, further comprises
- receiving source position data indicating at least one source position of the at least one source of radioactive radiation driven by the at least one source wire; and
- receiving measurement position data indicating at least one measurement position of the at least one measurement device driven by the at least one measurement wire.

8. The system according to claim 7, wherein
- at least one source tracking device is arranged in close proximity to the at least one source, wherein the source position data is derived from source tracking information obtained by the at least one source tracking device; and
- at least one measurement tracking device is arranged in close proximity to the at least one measurement device, wherein the measurement position data is derived from measurement tracking information obtained by the at least one measurement tracking device.

9. The system according to claim 7, wherein the controlling by the control unit further comprises
- receiving the radiation data measured by the at least one measurement device at the at least one measurement position; and
- deriving, based on the radiation data, dose distribution data of the dose delivered by the at least one source of radioactive radiation.

10. The system according to claim 9, wherein the controlling, by the control unit, further comprises
- comparing the dose distribution data with respective predicted dose distribution data predicted based on a therapy plan; and
- triggering at least one indication if the dose distribution data and the predicted dose distribution data deviate from one another by a pre-defined tolerance.

11. The system according to claim 1, wherein the controlling, by the control unit, further comprises
optimizing at least one measurement position of the at least one measurement wire on the basis of predicted dose distribution data obtained by predicting a predicted dose distribution based on a therapy plan.

12. The system according to claim 11, wherein the optimizing the at least one measurement position further comprises
- identifying a plurality of candidate positions for the measurement device;
- predicting, based on the therapy plan, the predicted dose distribution for each of the plurality of candidate positions in order to obtain the predicted dose distribution data; and
- determining, from the plurality of candidate positions, the at least one measurement position using a penalty method applied to the predicted dose distribution data.

13. A method for brachytherapy treatment, the method comprising the steps of:
- driving, by a drive unit controlled by a control unit, a plurality of drive wires inside a plurality of guiding tubes, the plurality of drive wires comprising at least one source wire for driving at least one source of radioactive radiation and at least one measurement wire for driving at least one measurement device for measuring radiation data; wherein
- the drive unit is configured to drive the at least one source wire and the at least one measurement wire independently from each other.

14. A computer program for controlling a system according to anyone of claims 1 to 12, which, when executed by a processing unit, is adapted to perform the method according to claim 13.

15. A computer-readable medium having stored thereon the computer program according to claim 14.
